# EUROPÄISCHE PATENTSCHRIFT

(11) **EP 0 356 679 B1**
(45) Veröffentlichungstag und Bekanntmachung des Hinweises auf die Patenterteilung: **22.06.1994**
(21) Anmeldenummer: 89113040.3
(22) Anmeldetag: 15.07.1989
(51) Int. Cl.: A61M 5/50, A61M 5/28

(54) **Einweg-Injektionsspritze**
One-way injection syringe
Seringue d'injection à usage unique

(30) Priorität: 12.08.1988 DE 3827335
(43) Veröffentlichungstag der Anmeldung: 07.03.1990
(73) Patentinhaber: Ing. Erich Pfeiffer GmbH & Co. KG, 78315 Radolfzell (DE)
(72) Erfinder: Fuchs, Karl-Heinz, D-7760 Radolfzell (DE)
(74) Vertreter: Patentanwälte Ruff, Beier, Schöndorf und Mütschele

(56) Entgegenhaltungen:
- EP-A- 0 170 181
- EP-A- 0 227 890
- FR-A- 1 314 002
- US-A- 4 475 906

## Beschreibung

Die Erfindung betrifft eine Einweg-Injektionsspritze nach dem Oberbegriff des Patentanspruches 1.

Durch die EP-A- 0 227 890 ist eine solche Injektionsspritze bekannt, bei der ein buchsenförmiger Kanülenträger so auf die Außenseite eines Endstutzens eines Zylinder-Behälters aufsetzbar ist, daß der Kanülenträger mit seiner Innenseite in Eingriff mit dem Behälter steht. Diese Verbindung bedarf hoher Genauigkeiten und der Verbindungsteil des Behälters muß formsteif sein.

Eine von einem Behälter nach außen durch eine Injektionskanüle ausstoßende Austragvorrichtung zeigt die US-A-4 475 906, jedoch ist hier der Austrag- bzw. Kanülenkanal unmittelbar an Wandungsteilen des Behälters ohne Zwischenschaltung einer Kanülenträgers befestigt, die auch ein Gehäuse eines federbelasteten Rückschlagventiles bilden. Dadurch ergeben sich unsichere Verbindungen, Beschädigungsgefahren und eine ergonomisch ungünstige Handhabbarkeit.

Nach der EP-A-0 170 181 ist eine Füllvorrichtung für Ballons und somit eine Austragvorrichtung bekannt, die im Vergleich zu der zuvor erläuterten Austragvorrichtung genau in der entgegengesetzten Richtung arbeitet, nämlich nicht aus dem durch den Ballon gebildeten Behälter austrägt, sondern in diesen einträgt. Diese Vorrichtung weist zwar ein in den Hals des Ballons eingesetztes Gehäuse mit Kanülen auf, jedoch muß dieses Gehäuse einschließlich der Kanülen aus dem Hals entfernt werden, wenn der Inhalt des Ballons durch den Hals ausgestoßen werden soll.

Außer einer einfachen Handhabbarkeit besteht auch mehr und mehr das Bedürfnis, Injektionsspritzen, insbesondere Injektionskanülen, die für den einmaligen Gebrauch bestimmt sind, so auszubilden, daß sie nach ihrem bestimmungsgemäßen Gebrauch nicht ein weiteres Mal wiederverwendet werden können. Insbesondere solche Injektionsspritzen, bei welchen die Injektionskanüle mit dem gefüllten Behälter für die Injektionsflüssigkeit bereits eine vorgefertigte Baueinheit bildet, sollen für die Wiederverwendung ungeeignet sein. Dieses Erfordernis ist bei bekannten Injektionsspritzen nicht oder höchstens mangelhaft erfüllt.

Der Erfindung liegt die Aufgabe zu Grunde, eine Injektionsspritze bzw. eine Injektionskanüle der genannten Art zu schaffen, bei welcher Nachteile bekannter Lösungen vermieden sind und die insbesondere auf einfache Weise gewährleistet, daß nach ihrem bestimmungsgemäßen Gebrauch eine nochmalige Verwendung zwangsläufig verhindert ist.

Zur Lösung dieser Aufgabe sind die Merkmale des Patentanspruches 1 geeignet. Es kann also eine Einweg-Injektionsspritze geschaffen werden, die eine an einen Folienbehälter oder einen ähnlichen Behälter für die Injektionsflüssigkeit angeschlossene Injektionskanüle aufweist, wobei dieser Injektionskanüle ein gegen Rücksaugen in Richtung zum Behälter selbsttätig sperrendes Ventil zugeordnet sein kann. Da Spritzen, deren Injektionsflüssigkeits-Kammer unlösbar mit der Injektionskanüle verbunden sind, nur durch diese Injektionskanüle hindurch wieder vollgesaugt und dadurch gefüllt werden können, wird durch die erfindungsgemäße Ausbildung eine Wiederfüllung der Kammer ohne Zerstörung der Injektionsspritze mit Sicherheit vermieden. Die Injektionskanüle selbst hat an ihrem von der Spitze abgekehrten Ende selbst keine Anschlußmuffe zur lösbaren Verbindung mit Injektionspumpen, so daß sie auch an anderen, beispielsweise nach Art von Schubkolbenpumpen ausgebildeten Injektionsbehältern nicht mehr angebracht werden kann und somit von einer Verwendung im Zusammenhang mit solchen Behältern ebenfalls ausgeschlossen ist.

Um eine besonders einfache Ausbildung zu erreichen, kann das Ventil als insbesondere federfreies Rückschlagventil ausgebildet sein, dessen Ventilkörper zwischen einer Schließstellung und einer Offenstellung frei beweglich innerhalb einer Ventilkammer angeordnet ist. Es ist aber auch denkbar, das Ventil so auszubilden, daß es am Ende des einmaligen Injektionsvorganges zwangsläufig, beispielsweise durch Verstopfen der Injektionskanüle, geschlossen wird.

Das Ventil könnte insofern auch innerhalb der Injektionskanüle vorgesehen bzw. baulich mit dieser vereinigt sein. Zweckmäßig jedoch ist das Ventil außerhalb der Injektionskanüle, insbesondere unmittelbar benachbart zu deren Einlaßseite vorgesehen, so daß Injektionskanülen verwendet werden können, die lediglich durch Rohrabschnitte gebildet sind.

In weiterer Ausgestaltung der Erfindung weist das Ventil einen scheibenförmigen, in einem Ventilgehäuse beweglichen bzw. bewegbaren Ventilkörper auf, wobei zweckmäßig das Ventilgehäuse bzw. der Ventilsitz aus einem thermoplastischen Polyester bzw. der Ventilkörper aus einem Polyurethan besteht. Die erfindungsgemäße Ausbildung ist auch für relativ feine Injektionsnadeln mit einem Durchmesser von beispielsweise etwa 5/10 mm geeignet, wobei das Ventilgehäuse zweckmäßig eine Außenweite hat, die höchstens dem 9- oder 10-fachen des Kanülen-Außendurchmessers entspricht, vorzugsweise demgegenüber kleiner ist. Die Axialerstreckung des Ventilgehäuses kann wiederum etwa in der Größenordnung seiner Außenweite oder geringfügig darüber liegen. Die Axialerstreckung des Ventilkörpers dagegen liegt zweckmäßig lediglich etwa in der Größenordnung des Außendurchmessers der Injektionskanüle.

Besonders vorteilhaft ist es, wenn der Schließsitz des Ventiles durch einen Stirndeckel eines Gehäuses, nämlich insbesondere des Ventilgehäuses, gebildet ist. Dieser Stirndeckel ist zweckmäßig hülsenförmig und in einen muffenförmigen Endteil des Gehäuses im wesentlichen unlösbar eingesetzt, beispielsweise eingesprengt, so daß er für eine Füllung des Behälters bzw. für eine Herausnahme des Ventilkörpers oder eine andersartige Öffnung des Ventiles nicht entfernt werden kann.

Die genannten Vorteile werden noch weiter dadurch verbessert, daß der Stirndeckel im wesentlichen vollständig innerhalb des Gehäuses liegt, vorzugweise mit dessen zugehöriger Stirnseite im wesentlichen bündig bzw. lückenfrei abschließt und dadurch keine Möglichkeiten bietet, ein Werkzeug zum Zwecke seiner Entfernung anzusetzen. Der Ventildeckel kann ebenfalls aus einem thermoplastischen Polyester, beispielsweise demselben Werkstoff wie das übrige Ventilgehäuse, bestehen, wobei es denkbar ist, den Ventilgehäusedeckel und das Ventilgehäuse haftend, insbesondere durch thermoplastische Verschweißung, innig so zu verbinden, daß selbst sorgfältigste Versuche einer Öffnung scheitern.

Eine sichere Verbindung zwischen Gehäuse und Stirndeckel ist auch zu erreichen, wenn letzterer einen erweiterten Bund aufweist, der in eine entsprechende Innennut des Gehäuses eingreift und im Abstand von der äußeren Stirnseite des Stirndeckels liegt, so daß er von außen nicht erreichbar ist. Dieser Bund kann z.B. anschließend an die innere Stirnseite des Stirndeckels vorgesehen sein und höchstens etwa über die Hälfte der Axialerstreckung des Stirndeckels reichen.

Eine Einlaßöffnung des Ventilgehäuses liegt zweckmäßig an dem von der Injektionskanüle abgekehrten Ende des Ventilgehäuses, wobei sie vorteilhaft durch den Stirndeckel selbst gebildet ist und in dessen Zentrum liegt. Die Axialerstreckung des Stirndeckels kann etwa in der Größenordnung der Hälfte der Axialerstreckung des Ventilgehäuses liegen, während seine Außenweite, insbesondere die des Bundes, höchstens um ein Viertel kleiner als die Außenweite des Ventilgehäuses ist.

Um eine leichtgängige Lagerung des Ventilkörpers zu gewährleisten, ist dieser in einem verrippten Ventilraum bewegbar gelagert, wobei vorzugsweise Axialrippen zur radialen Führung und Radialrippen zur axialen Anschlagbegrenzung des Ventilkörpers in der Offenstellung vorgesehen sind. Die Enden der Radialrippen umgeben zweckmäßig die Einlaßseite der Injektionskanüle, während ihre Seitenflächen etwa radiale, radial nach innen kreissegmentförmig verengte Strömungskanäle begrenzen, über welche die Injektionsflüssigkeit, von den Axialrippen entlang des Außenumfanges des Ventilkörpers kommend, unmittelbar in das Einlaßende der Injektionskanüle einströmen kann.

Um eine sichere Verankerung der Injektionskanüle sowie günstige Strömungsverhältnisse zu gewährleisten, schließt das Einlaßende der Injektionskanüle bündig mit einer inneren Stirnfläche des Gehäuses ab, über welche vorzugsweise die Radialrippen einteilig vorstehen. Jede Radialrippe liegt vorteilhaft symmetrisch zu einer Axialebene einer zugehörigen Axialrippe, in welche sie winkelförmig einteilig übergeht und die bis zum Stirndeckel reicht. Jede Radialrippe hat vorteilhaft gleiche Breite wie die zugehörige Axialrippe, wobei die Breite zweckmäßig etwa in der Größenordnung von dem 3- bis 4-fachen der Innenweite des Ventilraumes bzw. dem 2- bis 3-fachen der Außenweite des Ventilkörpers liegt und vier Axial- bzw. Radialrippen gleichmäßig über den Umfang verteilt sind.

Durch die beschriebene Ausbildung bildet das Ventilgehäuse unmittelbar einen Nadelträger für die Injektionskanüle, die ausschließlich über das Ventilgehäuse an dem Behälter anzubringen ist. Da das von der Injektionskanüle abgekehrte Ende des Ventilgehäuses eine im wesentlichen durchgehend ebene Stirnfläche bildet und somit für Steck- oder ähnliche Verbindungen ungeeignet ist und weil die Innenweite des Stirndeckels mit etwa 1 mm nur annähernd doppelt so groß wie die Außenweite der Injektionskanüle ist, ist die so gebildete Einheit aus Injektionskanüle und Nadelträger für die Verbindung mit anderen Injektionspumpen ungeeignet.

Zwar ist es denkbar, die Injektionskanüle einschließlich des Nadelträgers bzw. des Ventiles so auszubilden, daß sie an eine kolbenpumpenartige Injektionspumpe angeschlossen bzw. mit dieser unlösbar baulich vereinigt werden kann. Besonders vorteilhaft ist es jedoch, wenn als Behälter ein Folienbehälter aus Kunststoff-Folie, Aluminium-Folie oder dgl. verwendet wird, der zweckmäßig aus zwei aufeinanderliegenden und entlang des gemeinsamen Randes miteinander verschweißten Folienteilen hergestellt ist. Vorteilhaft ist das von der Kanülenspitze abgekehrte Ende der Injektionskanüle durch Ummantelung bzw. Einschweißung an dem Folienbehälter befestigt, wobei diese Ummantelung bevorzugt vor allem für das Ventilgehäuse bzw. den Nadelträger derart vorgesehen ist, daß dieser vollständig zwischen den Folienteilen liegt und von diesen abgedeckt ist. Der Behälter kann mit dem Ventilgehäuse bzw. dem Nadelträger durch Verschweißung unlösbar verbunden sein, wobei die Verschweißung zweckmäßig im wesentlichen ununterbrochen über den gesamten Umfang des Ventilgehäuses, an dessen dem Behälterinneren zugekehrten Stirnseite, der davon abgekehrten Stirnseite und zweckmäßig auch jeder weiteren Stirnseite vorgesehen ist, die beispielsweise durch eine abgesetzte Außenform des Ventilgehäuses gebildet ist. Es ist denkbar, im Zuge dieser Verschweißung auch gleichzeitig die Verschweißung zwischen Ventilgehäuse und Stirndeckel vorzunehmen.

Gemäß der Erfindung ist es des weiteren denkbar, ein Ventil vorzusehen, welches das Behälterinnere gegenüber der Injektionskanüle bis zum bestimmungsgemäßen Verbrauch geschlossen hält und dann beispielsweise durch Erhöhung des Innendruckes im Behälter oder eine ähnliche Maßnahme irreversibel oder aber wiederverschließbar geöffnet werden kann. Dieses Ventil könnte z.B. durch das beschriebene Sicherungs- bzw. Rückschlagventil gebildet sein, wobei dann der Ventilkörper in seiner Schließstellung ausrastbar kraftschlüssig gehalten sein könnte. Besonders vorteilhaft ist es jedoch, wenn als Ventil ein Verschluß, insbesondere ein Aufreißverschluß vorgesehen ist, welcher in Ausströmrichtung vor dem Sicherungs- bzw. Rückschlagventil unmittelbar benachbart zur Einlaßseite des Ventilgehäuses liegt. Dieser Verschluß kann in besonders einfacher Weise durch eine Zone gebildet werden, in welcher die beiden Folienteile des Folienbehälters mit einer reduzierten Sollbruch-Haftkraft aneinander befestigt sind, so daß aufgrund einer vorbestimmten Erhöhung des Innendruckes des Folienbehälters durch Zusammenpressen von dessen Folienteilen der Verschluß von selbst öffnet und die Leitungsverbindung vorbei am Ventil durch das Ventilgehäuse zur Injektionskanüle freigibt.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Ein Ausführungsbeispiel der Erfindung ist in den Zeichnungen dargestellt und wird im folgenden näher erläutert. In den Zeichnungen zeigen:
- Fig. 1: eine erfindungsgemäße Einweg-Injektionsspritze, teilweise im Axialschnitt,
- Fig. 2: die Injektionsspritze gemäß Fig. 1 in Ansicht und
- Fig. 3: einen Ausschnitt der Fig. 1 in wesentlich vergrößerter und axial geschnittener Darstellung.

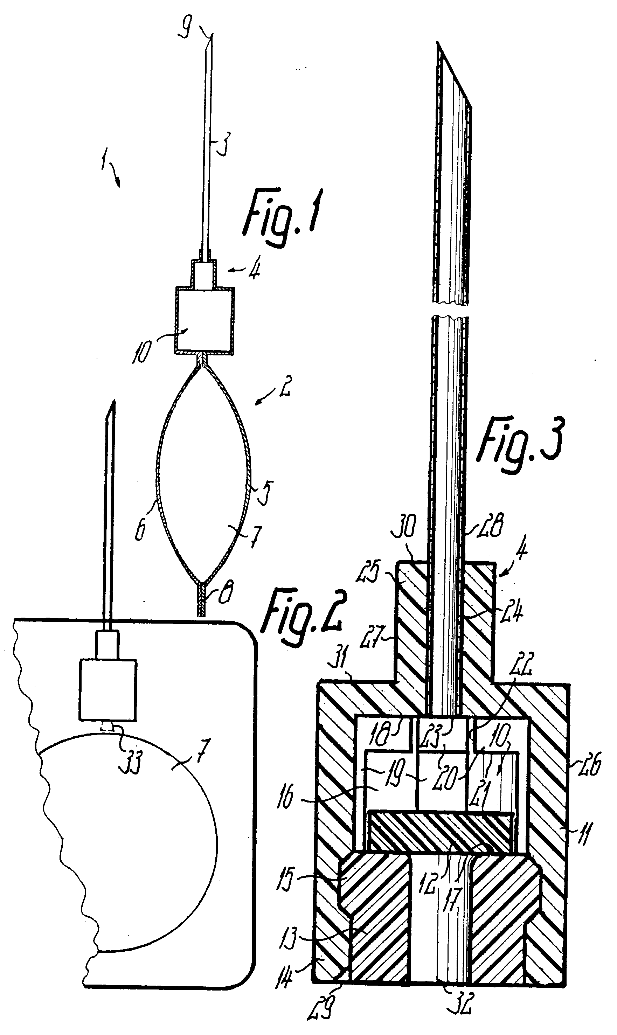

Die Einmal-Injektionsspritze 1 gemäß den Figuren 1 bis 3 weist einen Folienbehälter 2 und eine mit diesem über einen Nadelträger 4 baulich vereinigte Injektionskanüle 3 auf. Der Folienbehälter 2 besteht aus zwei aufeinanderliegenden rechteckigen bzw. annähernd quadratischen Folienteilen 5, 6, die innerhalb ihrer gemeinsamen Randkanten eine kissenförmig gefüllte, in Ansicht annähernd kreisförmige Behälterkammer 7 umschließen und vom Außenumfang dieser Behälterkammer 7 bis zu ihren Randkanten durch eine ganzflächige Schweißverbindung unlösbar miteinander verbunden sind. Die Injektionskanüle 3 steht über eine Außenkante, und zwar etwa in der Mitte von deren Breite rechtwinklig vor, wobei die Behälterkammer 7 von dieser Randkante weiter entfernt ist als von der davon abgekehrten und parallelen Randkante. Am äußeren, im Abstand von dem Folienbehälter 2 liegenden Ende weist die Injektionskanüle 3 eine Kanülenspitze 9 auf.

Der Injektionskanüle 3 ist ein Ventil 10 in Form eines Rückschlagventiles zugeordnet, das ein annähernd zylindrisches Ventilgehäuse 11 aufweist, welches einteilig mit dem Nadelträger 4 ausgebildet ist. Das Ventilgehäuse 11, in welchem ein kreisscheibenförmiger Ventilkörper 12 frei beweglich angeordnet ist, ist im wesentlichen durch einen hülsen- bzw. napfförmigen, zur Injektionskanüle 3 koaxialen Bauteil gebildet, in dessen von der Injektionskanüle 3 abgekehrten Endteil 14 ein hülsenförmiger Stirndeckel 13 vollständig versenkt eingesetzt ist. Dieser Stirndeckel 13 weist an seinem inneren Ende einen um wenige zehntel Millimeter über seinen ansonsten zylindrischen Außenumfang vorstehenden Bund 15 auf, der in eine eng an ihn angepaßte Ringnut an der Innenumfangsfläche des Ventilgehäuses 11 mit Vorspannung eingreift. Die Innenumfangsfläche des Ventilgehäuses 11 weist im Bereich der von diesem begrenzten Ventilkammer 16 bis zu der von der Injektionskanüle 3 abgekehrten Stirnseite durchgehend im wesentlichen konstante Querschnitte auf, die lediglich durch die genannte innere Ringnut unterbrochen sind. Außerdem kann diese Innenumfangsfläche an der genannten Stirnseite mit einer phasenartigen, konischen Erweiterung versehen sein, die eine Einführfläche beim Einsprengen des Stirndeckels 13 bildet. Stattdessen oder zusätzlich hierzu kann aber auch nur am zugehörigen inneren Ende des Stirndeckels 13 bzw. des Ringbundes 15 eine solche Einführfläche vorgesehen sein.

Die innere, ebene und ringscheibenförmige Stirnfläche des Stirndeckels 13 bildet den Ventilsitz 17 für die ebene Ventilschließfläche des Ventilkörpers 12. Die Außenweite des Ventilkörpers 12 ist kleiner als die Innenweite des Ventilgehäuses 11. Dem Ventilsitz 17 gegenüberliegend bildet die zugehörige Stirnwand 18 des Ventilgehäuses 11 den Boden der Ventilkammer, wobei die Injektionskanüle 3 diesen Boden durchsetzt.

Zur Führung des Ventilkörpers 12 sind an der Innenumfangsfläche des Ventilgehäuses 11 einteilig mit diesem ausgebildete, verhältnismäßig niedrige bzw. bandförmige Axialrippen 19 vorgesehen, welche bis zur inneren Stirnfläche des Stirndeckels 13 reichen und somit bei jeder Stellung des Ventilkörpers 12 nur mit dem notwendigen Bewegungsspiel unmittelbar benachbart zum Außenumfang des Ventilkörpers 12 liegen. An ihren vom Ventilsitz 17 abgekehrten Enden gehen die Axialrippen 19 in Radialrippen 20 über, die größere Rippenhöhe haben und deren dem Ventilsitz 17 zugekehrte, in einer gemeinsamen Ebene liegende Kopfkanten einen Anschlag für die Offenstellung des Ventilkörpers 17 bilden. Die einander bzw. der Mittelachse der Injektionskanüle 3 zugekehrten und im wesentlichen ebenen Enden 22 der Radialrippen 20 umgeben die Einlaßseite 23 der Injektionskanüle 3, deren innere Endfläche bündig mit der inneren Stirnfläche der Stirnwand 18 abschließt. Der Radialabstand der Enden 22 von der Mittelachse der Einlaßseite 23 der Injektionskanüle 3 ist größer als deren halber Durchmesser, so daß sich günstige Strömungsverhältnisse ergeben und selbst dann noch zwischen benachbarten Enden 22 Durchtrittsschlitze frei bleiben, wenn die Radialrippen 20 verhältnismäßig breit ausgebildet sind.

Das von der Kanülenspitze 9 abgekehrte Ende der zweckmäßig metallischen Injektionskanüle 3 bildet einen Schaftteil 24, der in dem Nadelträger 4 durch Einspritzen unlösbar verankert ist und bis an die zugehörige Stirnfläche der Ventilkammer 16 reicht. Der Nadelträger 4 ist durch einen Gehäuseansatz 25 des Ventilgehäuses 11 gebildet, der einteilig an die Stirnwand 18 anschließt, ausschließlich nach außen vorsteht und eine Außenweite hat, die kleiner als die Hälfte der Außenweite des Ventilgehäuses 11 bzw. etwa 3-fach so groß wie der Außendurchmesser der Injektionskanüle 3 ist. Die Axialerstreckung des Ansatzes 25 ist größer als die Dicke der Stirnwand 18 sowie geringfügig größer als seine Außenweite.

Durch die beschriebene Ausbildung sind drei aneinanderschließende, im Außendurchmesser zweifach abgesetzte zylindrische Umfangsflächen 26, 27, 28 einerseits durch das Ventilgehäuse 11 und andererseits durch den Ansatz 25 sowie durch den über diesen vorstehenden Teil der Injektionskanüle 3 gebildet. Des weiteren sind drei zueinander im wesentlichen parallele Stirnflächen 29, 30, 31 gebildet, von denen eine durch die bündig miteinander abschließenden Stirnflächen des Endteiles 14 des Ventilgehäuses 11 und des Stirndeckels 16 gebildet ist, während eine ringförmige Stirnfläche 31 durch den Übergang zwischen dem Ventilgehäuse 11 und dem Ansatz 25 gebildet ist, der seinerseits eine an die Umfangsfläche 28 der Injektionskanüle 3 anschließende, ringförmige Stirnfläche 30 bildet. Die beiden Folienteile 5, 6 sind an diesen Umfangsflächen 26, 27, 28 sowie an den Stirnflächen 29, 30, 31 durch Schweißung so befestigt, daß jeder Folienteil etwa die Hälfte jeder Umfangsfläche sowie die Hälfte jeder Stirnfläche bedeckt und dadurch das Ventilgehäuse 11 sowie der Nadelträger 4 und ein Teil der Injektionskanüle 3 vollständig ummantelt sind.

Die innere Stirnfläche 29 des Ventilgehäuses 11 liegt dabei mit geringem Abstand von der Behälterkammer 7. In dieser Stirnfläche 29 ist die Einlaßöffnung 32 für das Ventil 10 vorgesehen, die zweckmäßig durch den Zentralkanal des Stirndeckels 13 gebildet ist. Zwischen dieser Einlaßöffnung 32 und der Behälterkammer 7 bilden die beiden Folienteile 5, 6 einen Verschluß 33, der durch hydraulischen Druck dadurch zu öffnen ist, daß in seinem Bereich die beiden Folienteile 5, 6 nur mit einer reduzierten Haftkraft miteinander verbunden sind und beim Lösen voneinander einen genau begrenzten Verbindungskanal zwischen der Behälterkammer 7 und der Einlaßöffnung 32 freigeben.

Durch die erfindungsgemäße Ausbildung kann die Injektionsspritze 1 sehr einfach gehandhabt werden, wobei für das Ausstoßen der Injektionsflüssigkeit lediglich die beiden Folienteile 5, 6 im Bereich der Behälterkammer 7 mit zwei Fingern einer Hand zusammengepreßt werden müssen. Eine Wiederfüllung der Behälterkammer 7 ist aufgrund des gegen Rücksaugung sofort schließenden Ventiles 10 ebenso ausgeschlossen, wie eine Luftrücksaugung durch die Injektionskanüle 3 in die Behälterkammer 7.

## Patentansprüche

1. Einweg-Injektionsspritze zum Austrag von Medien, mit einer Injektionskanüle (3), die über einen mit dem Behälter (2) verbundenen Kanülenträger (4) an den Behälter (2) angeschlossen ist, wobei der Kanülenträger (4) ein Gehäuse (11) mit Durchlaß für das Medium vom Behälter (2) zur Mündung (9) bildet,
dadurch gekennzeichnet,
daß der Kanülenträger (4) mit seiner Außenseite unlösbar mit dem Behälter (2) verbunden ist.

2. Injektionsspritze nach Anspruch 1, dadurch gekennzeichnet, daß der Kanülenträger (4) wenigstens teilweise von Wandungsteilen des Behälters (2) umgeben ist, die aus Folienwerkstoff bestehen und daß insbesondere der Kanülenträger (4) durch Einschweißung an dem Behälter (2) befestigt ist.

3. Injektionsspritze nach Anspruch 1 oder 2, dadurch gekennzeichnet,
daß der Behälter (2) an mindestens einer Stirnfläche (29, 30, 31) des Kanülenträgers (4) befestigt ist.

4. Injektionsspritze nach einem der Ansprüche 1 bis 3, dadurch gekennzeichnet, daß der Behälter (2) aus zwei flächig aneinander befestigten Folienteilen (5, 6) besteht, und daß der Kanülenträger (4) wenigstens an seiner dem Behälter (2) zugwandten Stirnfläche (29) eng anliegend abgedeckt ist.

5. Injektionsspritze nach einem der vorhergehenden Ansprüche, dadurch gekennzeichnet, daß
der Kanülenträger (4) am Umfang (26, 27, 28) ununterbrochen vollständig ummantelt zwischen den Folienteilen (5, 6) eingeschweißt ist und daß dabei ggf. der Behälter (2) auch die Injektionskanüle (3) auf einem Teil ihrer Länge umgibt.

6. Injektionsspritze nach einem der vorhergehenden Ansprüche, dadurch gekennzeichnet, daß in das Gehäuse (11) des Kanülenträgers (4) ein Einsatzkörper (13) gegen Entfernen, vorzugsweise durch einen Bund (15), gesichert eingesetzt ist, und daß insbesondere der Einsatzkörper (13) nach Art eines eine Einlaßöffnung (32) begrenzenden Pfropfens innerhalb eines muffenförmigen Endteiles (14) des Gehäuses (11) liegt.

7. Injektionsspritze nach einem der vorhergehenden Ansprüche, dadaurch gekennzeichnet, daß der an den Behälter (2) angeschlossenen Injektionskanüle (3) ein gegen Rücksaugen in Richtung zum Behälter (2) selbsttätig sperrendes Ventil (10) zugeordnet ist, das vorzugsweise als federfreies Rückschlagventil ausgebildet und in dem Kanülenträger (4) vorgesehen ist, wobei insbesondere ein Ventilteil des Ventiles (10) durch einen Einsatzkörper (13) gebildet ist.

8. Injektionsspritze nach Anspruch 7, dadurch gekennzeichnet, daß ein Ventilkörper (12) des Ventiles (10) in einer verrippten Ventilkammer (16) bewegbar gelagert ist, die zur Führung des Ventilkörpers (12) Axialrippen (19) und zur Anschlagbegrenzung des Ventilkörpers (12) in der Offenstellung Radialrippen (20) enthält, wobei vorzugsweise die Enden (22) der Radialrippen (20) die Einlaßseite (23) der Injektionskanüle (3) umgeben und mit ihren Seitenflächen etwa radiale Strömungskanäle begrenzen.

9. Injektionsspritze nach einem der vorhergehenden Ansprüche, dadurch gekennzeichnet, daß der Kanülenträger (4) im wesentlichen achssymmetrisch und insbesondere abgesetzt zylindrisch ausgebildet ist.

10. Injektionsspritze nach einem der vorhergehenden Ansprüche, dadurch gekennzeichnet, daß im Durchlaß ein unter Innendruck zu öffnender Verschluß (33) vorgesehen ist, der insbesondere als Aufreißverschluß ausgebildet ist.

11. Injektionsspritze nach Anspruch 2 und 10, dadurch gekennzeichnet, daß der Verschluß (33) durch eine Zone gebildet ist, in welcher die beiden Folienteile (5, 6) mit einer reduzierten Sollbruch-Haftkraft befestigt sind.

## Claims

1. Disposable injection syringe for the discharge of media, with an injection cannula (3), which is connected by means of a cannula holder (4) connected to the container (2) to the container (2), the cannula holder (4) forming a casing (11) with a passage for the medium from the container (2) to the opening (9), characterized in that the cannula holder (4) is non-detachably connected by its outside to the container (2).

2. Injection syringe according to claim 1, characterized in that the cannula holder (4) is at least partly surrounded by wall parts of the container (2), which are made from film material and that in particular the cannula holder (4) is fixed by welding to the container (2).

3. Injection syringe according to claim 1 or 2, characterized in that the container (2) is fixed to at least one face (29, 30, 31) of the cannula holder (4).

4. Injection syringe according to one of the claims 1 to 3, characterized in that the container (2) comprises two flat fixed together film parts (5, 6) and that the cannula holder (4) is covered in closely engaging manner at least on its face (29) facing the container (2).

5. Injection syringe according to one of the preceding claims, characterized in that the cannula holder (4) is continuously completely enveloped on the circumference (26, 27, 28) and is welded between the film parts (5, 6) and that optionally the container (2) also surrounds the injection cannula (3) over part of its length.

6. Injection syringe according to one of the preceding claims, characterized in that in the casing (11) of the cannula holder (4) is inserted an insert body (13) to prevent removal preferably secured by a collar (15), and that in particular the insert body (13) is located in the manner of a plug defining an inlet opening (32) within a muff-like end part (14) of the casing (11).

7. Injection syringe according to one of the preceding claims, characterized in that the injection cannula (3) connected to the container (2) has associated with it a valve (10) which automatically locks against back suction towards the container (2) and which is preferably constructed as a spring-free check valve and is provided in the cannula holder (4) and in particular one valve part of the valve (10) is formed by an insert body (13).

8. Injection syringe according to claim 7, characterized in that a valve body (12) of the valve (10) is movably mounted in a ribbed valve chamber (16), which has for the guidance of the valve body (12) axial ribs (19) and for the stop limitation of the valve body (12) in the open position radial ribs (20), preferably the ends (22) of the radial ribs (20) surrounding the inlet side (23) of the injection cannula (3) and bounding with their lateral faces radial flow channels.

9. Injection syringe according to one of the preceding claims, characterized in that the cannula holder (4) is substantially axially symmetrical and is in particular offset cylindrical.

10. Injection syringe according to one of the preceding claims, characterized in that in the passage is provided a closure (33) to be opened under internal pressure and which in particular is constructed as a tear-open closure.

11. Injection syringe according to claim 2 and 10, characterized in that the closure (33) is formed by a zone in which the two film parts (5, 6) are fixed with a reduced predetermined breaking adhesion.

## Revendications

1. Seringue d'injection à usage unique pour administrer des substances, présentant une canule d'injection (3) raccordée au réceptacle (2) par l'intermédiaire d'un porte-canule (4) relié audit réceptacle (2), le porte-canule (4) formant un boîtier (11) ménageant, pour la substance, un passage du réceptacle (2) jusqu'à l'embouchure (9),
caractérisée par le fait
que le porte-canule (4) est relié au réceptacle (2) par sa face extérieure, de manière indissociable.

2. Seringue d'injection selon la revendication 1, caractérisée par le fait que le porte-canule (4) est entouré, au moins en partie, par des régions de paroi du réceptacle (2) qui consistent en un matériau stratifié ; et par le fait que le porte-canule (4) est notamment fixé au réceptacle (2) par soudage intégré.

3. Seringue d'injection selon la revendication 1 ou 2, caractérisée par le fait
que le réceptacle (2) est fixé à au moins une face extrême (29, 30, 31) du porte-canule (4).

4. Seringue d'injection selon l'une des revendications 1 à 3, caractérisée par le fait que le réceptacle (2) se compose de deux parties (5, 6) constituées par des feuilles et fixées à plat l'une à l'autre ; et par le fait que le porte-canule (4) est obturé, avec contact intime, au moins à sa face extrême (29) tournée vers le réceptacle (2).

5. Seringue d'injection selon l'une des revendications précédentes,
caractérisée par le fait que
le porte-canule (4) est intégré par soudage entre les parties en feuilles (5, 6), avec enrobage intégral ininterrompu sur la périphérie (26, 27, 28) ; et par le fait que, le cas échéant, le réceptacle (2) entoure alors également la canule d'injection (3) sur une partie de sa longueur.

6. Seringue d'injection selon l'une des revendications précédentes, caractérisée par le fait qu'une pièce prisonnière (13) est insérée de manière indissociable dans le boîtier (11) du porte-canule (4), de préférence par l'intermédiaire d'un collet (15) ; et par le fait que la pièce prisonnière (13) est en particulier logée, à la manière d'un bouchon délimitant un orifice d'admission (32), à l'intérieur d'une partie extrême (14) du boîtier (11) qui est configurée en un manchon.

7. Seringue d'injection selon l'une des revendications précédentes, caractérisée par le fait qu'une soupape (10), affectée à la canule d'injection (3) raccordée au réceptacle (2), et exerçant un effet de blocage automatique empêchant une rétroaspiration en direction dudit réceptacle (2), est de préférence réalisée sous la forme d'un clapet antiretour dépourvu de ressort et est prévue dans le porte-canule (4), une partie obturatrice de la soupape (10) étant notamment formée par une pièce prisonnière (13).

8. Seringue d'injection selon la revendication 7, caractérisée par le fait qu'un corps obturateur (12) de la soupape (10) est monté mobile dans une chambre d'obturation (16) nervurée, qui renferme des nervures axiales (19) pour guider le corps obturateur (12), et des nervures radiales (20) pour arrêter ledit corps obturateur (12) par effet de butée dans la position ouverte, les extrémités (22) des nervures radiales (20) entourant de préférence le côté admission (23) de la canule d'injection (3) et délimitant, par leurs surfaces latérales, des canaux de circulation sensiblement radiaux.

9. Seringue d'injection selon l'une des revendications précédentes, caractérisée par le fait que le porte-canule (4) est pour l'essentiel réalisé avec symétrie axiale et, en particulier, de configuration cylindrique étagée.

10. Seringue d'injection selon l'une des revendications précédentes, caractérisée par le fait qu'une obturation (33), conçue pour être ouverte sous l'effet d'une pression intérieure, est prévue dans le passage et est notamment réalisée sous la forme d'une obturation défonçable.

11. Seringue d'injection selon les revendications 2 et 10, caractérisée par le fait que l'obturation (33) est formée par une zone dans laquelle les deux parties en feuilles (5, 6) sont fixées avec une force réduite de retenue à rupture programmée.
